(19) Europäisches Patentamt
European Patent Office
Office européen des brevets

(11) Numéro de publication: **0 138 714**
**B1**

# (12) FASCICULE DE BREVET EUROPÉEN

(45) Date de publication du fascicule du brevet: **20.05.87**

(21) Numéro de dépôt: **84402064.4**

(22) Date de dépôt: **12.10.84**

(51) Int. Cl.4: **C 07 C 103/44,** C 07 D 295/10, A 61 K 31/165 // C07C103/42

## (54) Dérivés de 1-(acétylaminophényl)-2-amino-propanone, leur utilisation en thérapeutique et leur procédé de préparation.

(30) Priorité: **14.10.83 FR 8316408**
**20.08.84 FR 8412963**

(43) Date de publication de la demande:
**24.04.85 Bulletin 85/17**

(45) Mention de la délivrance du brevet:
**20.05.87 Bulletin 87/21**

(84) Etats contractants désignés:
**AT BE CH DE FR GB IT LI LU NL SE**

(56) Documents cité:
**CH-A-348 240**
**FR-A-1 463 813**

**CHEMICAL ABSTRACTS, vol. 87, no. 5, 1er août 1977, page 527, no. 39533x, Columbus, Ohio, US; & JP - A - 76 141 831 (YOSHITOMI PHARMACEUTICAL INDUSTRIES LTD.) 07-12-1976**

(73) Titulaire: **LABORATOIRE L. LAFON Société anonyme dite:, 1 rue Georges Médéric, F-94701 Maisons- Alfort (FR)**

(72) Inventeur: **Lafon, Louis, 5 rue de l'Alboni, F-75016 Paris (FR)**

(74) Mandataire: **Clisci, Serge, S.A. FEDIT- LORIOT CONSEILS EN PROPRIETE INDUSTRIELLE 38, avenue Hoche, F-75008 Paris (FR)**

**EP 0 138 714 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention a trait à de nouveaux dérivés de 1-(acétylaminophényl)-2-amino-propanone. Elle concerne également l'utilisation de ces nouveaux dérivés en thérapeutique, notamment en tant qu'agents antidépresseurs du système nerveux central (SNC), d'une part, et leur procédé de préparation, d'autre part.

On préconise donc de nouveaux dérivés de 1-(acétylaminophényl)-2-amino-propanone qui sont utiles en thérapeutique. Ces nouveaux dérivés agissent tous sur le SNC, en particulier en tant qu'agents antidépresseurs. A côté des propriétés antidépressives communes à l'ensemble on constate que ces dérivés présentent des effets stimulants (ou excitants) sur le SNC. Par ailleurs certains de ces dérivés possèdent des effets immunologiques et/ou cardiovasculaires qui sont bénéfiques comme indiqué ci-après.

Les nouveaux dérivés de 1-(acétylaminophényl)-2-amino-propanone que l'on préconise ici sont caractérisés en ce qu'ils sont choisis parmi l'ensemble constitué par

a) les composés de formule

Ar - CO - CH ($CH_3$) - $NR_1R_2$ (I)

dans laquelle,

- $R_1$ représente un groupe alkyle en $C_1$-$C_4$, ou un groupe cycloalkyle en $C_3$-$C_6$;

- $R_2$ représente l'atome d'hydrogène ou un groupe alkyle en $C_1$-$C_4$;

- $R_1$ et $R^2$ considérés ensemble peuvent former avec l'atome d'azote auquel ils sont liés un groupe N - hétérocyclique de 5 à 7 sommets susceptible (i) de comporter un second hétéroatome notamment choisi parmi N, O et S, et (ii) d'être substitué, ledit groupe hétérocyclique $NR_1R_2$ étant notamment choisi parmi l'ensemble constitué par les groupes pyrrolidino, morpholino, thiomorpholino, pipéridino, hexaméthylèneimino, pipérazino, 4-méthyl-pipérazino, 4-(β-hydroxyéthyl)-pipérazino, 4-phénylpipérazino, 4-(p-chlorophényl)-pipérazino;

- Ar représente un groupe acétylaminophényle de formule

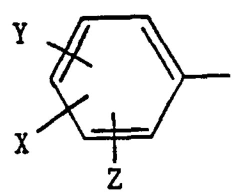

où X est $CH_3CONH$, et, Y et Z qui peuvent être identiques ou différents, représentent chacun un atome d'hydrogène ou d'halogène; et,

b) leurs sels d'addition.

Parmi les groupes alkyle en $C_1$-$C_4$ inclus dans les définitions des groupes $R_1$ et $R_2$ on peut notamment citer les groupes $CH_3$, $CH_2CH_3$, $CH_2CH_2CH_3$, $CH(CH_3)_2$, $C(CH_3)_3$, $CH(CH_3)CH_2CH_3$, $CH_2CH(CH_3)_2$ et $CH_2CH_2CH_2CH_3$.

Parmi les groupes cycloalkyle en $C_3$-$C_6$ inclus dans la définition du groupe $R_1$, on peut mentionner notamment les groupes cyclopropyle, cyclopentyle et cyclohexyle.

Les groupes N-hétérocycliques $NR_1R_2$ qui conviennent selon l'invention sont avantageusement saturés. Ils comprennent de 5 à 7 sommets, peuvent comporter un second hétéroatome choisi parmi l'ensemble constitué par N, O et S, et, peuvent être substitués notamment par des groupes alkyle en $C_1$-$C_4$, hydroxyalkyle en $C_1$-$C_4$ (notamment $CH_2CH_2OH$), aryle ou halogénoaryle (notamment phényle et 4-chlorophényle).

Parmi les atomes d'halogène inclus dans les définition des groupes Y et Z on peut notamment citer F, Cl et Br qui conviennent, l'atome d'halogène préféré étant ici Cl. Le groupe acétylamino X = $CH_3CONH$ peut être en position ortho, méta ou para, les positions méta et para sont les préférées. Les groupes Ar préférés sont les groupes 4-acétylaminophényle, 4-acétylamino-3-chlorophényle, 4-acétylamino-3,5-dichlorophényle, 3-acétylaminophényle et 3-acétylamino-4-chlorophényle.

Par sels d'addition, on entend ici les sels d'addition d'acide obtenus par réaction d'une des bases libres susvisées avec un acide minéral ou organique, d'une part, et les sels d'ammonium, d'autre part. Parmi les acides utilisables pour salifier les bases libres susvisées,on peut notamment mentionner les acides chlorhydrique, bromhydrique, acétique, formique, propionique, oxalique, fumarique, maléique, succinique, benzoïque, cinnamique, mandélique, citrique, malique, tartrique, aspartique, glutamique, méthanesulfonique, p-toluènesulfonique. Parmi les composés permettant d'obtenir des sels d'ammonium, on peut notamment citer $ICH_3$ et $ClCH_3$. D'une manière générale les sels d'addition d'acide sont préférés aux sels d'ammonium.

Un certain nombre de composés selon l'invention a été consigné de façon nullement limitative dans le tableau I ci-après. Les points de fusion qui ont été donnés sont des points de fusion instantanée déterminés au

banc Kofler.

TABLEAU I

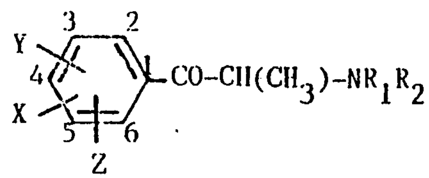

| Produit | N° de Code | X | Y | Z | $NR_1R_2$ | F(°C) |
|---|---|---|---|---|---|---|
| Ex1(a) | CRL 41 152 | 4-$CH_3CONH$ | H | H | $NHCH(CH_3)_2$ | 240 |
| Ex2(a) | CRL 41 177 | 4-$CH_3CONH$ | H | H | $NHC(CH_3)_3$ | (b) |
| Ex3(a) | CRL 41 192 | 4-$CH_3CONH$ | 3-Cl | H | $NHCH(CH_3)_2$ | 260 |
| Ex4(a) | CRL 41 212 | 4-$CH_3CONH$ | H | H | $NH(CH_2)_2CH_3$ | 230(c) |
| Ex5(a) | CRL 41 217 | 4-$CH_3CONH$ | H | H | $NHCH_3$ | ( d) |
| Ex6(a) | CRL 41 219 | 4-$CH_3CONH$ | 3-Cl | H | pyrrolidino | 260 |
| Ex7(a) | CRL 41 220 | 4-$CH_3CONH$ | 3-Cl | 5-Cl | $NHCH(CH_3)_2$ | (b) |
| Ex8(a) | CRL 41 224 | 4-$CH_3CONH$ | H | H | $NHCH_2CH_3$ | 230(c) |
| Ex9(a) | CRL 41 232 | 4-$CH_3CONH$ | H | H | $N(CH_3)_2$ | 252(c) |
| Ex10(a) | CRL 41 236 | 4-$CH_3CONH$ | H | H | morpholino | 255(c) |
| Ex11(a) | CRL 41 240 | 4-$CH_3CONH$ | H | H | pipéridino | 240(c) |

**TABLEAU I** (suite et fin)

| Produit | N° de Code | X | Y | Z | $NR_1R_2$ | F(°C) |
|---|---|---|---|---|---|---|
| Ex12(e) | CRL 41 242 | 4-$CH_3$CONH | H | H | 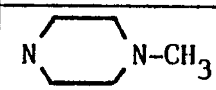 | 230(c) |
| Ex13(a) | CRL 41 245 | 4-$CH_3$CONH | H | H | 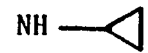 | 200(c) |
| Ex14(a) | CRL 41 247 | 4-$CH_3$CONH | H | H | 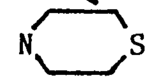 | 200- 210(c) |
| Ex15(a) | CRL 41 254 | 3-$CH_3$CONH | H | H | $NHCH(CH_3)_2$ | 250(c) |
| Ex16(a) | CRL 41 259 | 3-$CH_3$CONH | H | H | $N(CH_3)_2$ | 250(c) |
| Ex17(a) | CRL 41 262 | 3-$CH_3$CONH | H | H | 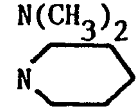 | 245(c) |
| Ex18(a) | CRL 41 267 | 3-$CH_3$CONH | H | H | 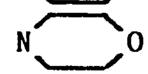 | 220(c) |
| Ex19(a) | CRL 41 269 | 3-$CH_3$CONH | H | H | 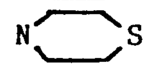 | 250 |
| Ex20(a) | CRL 41 277 | 3-$CH_3$CONH | H | H | $NH(CH_2)_2CH_3$ | 220(c) |

Notes

(a) : chlorhydrate ;
(b) : le point de fusion est supérieur à 260 °C ;
(c) : avec décomposition ;
(d) : le point de fusion est supérieur à 250 ° C ;
(e) : dichlorhydrate.

Notes
(a): chlorhydrate;
(b): le point de fusion est supérieur à 260 °C;
(c): avec décomposition;
(d): le point de fusion est supérieur à 250 ° C;
(e): dichlorhydrate.

Les nouveaux dérivés de 1 - (acétylaminophényl) - 2-amino - propanone peuvent être préparés selon une méthode connue en soi par application de mécanismes réactionnels classiques. On préconise, pour préparer ces dérivés, deux méthodes de synthèse schématisées par les réactions suivantes:

**Variante A:**

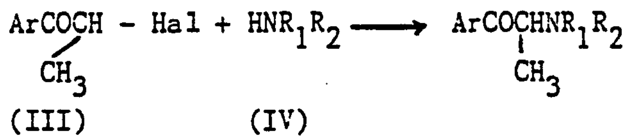

**Variante B:**

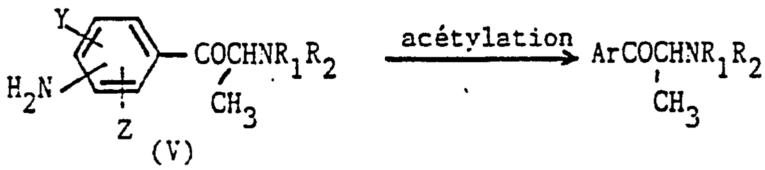


La variante A consiste à faire réagir un dérivé halogéné de formule III (ou Ar est défini comme indiqué ci-dessus et Hal représente un atome d'halogène, en particulier Cl ou Br et de préférence Cl pour le rendement) avec une amine de formule IV (où $R_1$ et $R_2$ sont définis comme indiqué ci-dessus).

De préférence on utilisera plus d'une mole de IV pour une mole de III, l'amine IV intervenant simultanément en tant que réactif et solvant ou co-solvant de la réaction. Ladite réaction de la 1 - (acétylaminophényl) - 2 - halogéno - propanone III avec l'amine IV est avantageusement effectuée selon la variante A pendant au moins 0,5 h à une température comprise entre 15°C et la température de reflux du milieu réactionnel.

La variante B consiste à soumettre un dérivé de 1 - (aminophényl) - 2 - amino - propanone de formule V (où Y, Z, $R_1$ et $R_2$ sont définis comme indiqué ci-dessus) à une réaction d'acétylation au moyen d'un agent d'acétylation en excès par rapport aux conditions stoechiométriques choisi parmi l'ensemble constitué par les halogénures d'acétyle (de préférence $CH_3COCl$ pour le rendement) et l'anhydride de l'acide acétique $(CH_3CO)_2O$. Les composés de formule V sont décrits dans la demande française N°8 412 962 du 20 août 1984 de la Demanderesse.

De façon avantageuse on fera réagir, selon la variante B, le dérivé de formule V dans $CH_3COCl$ pendant au moins 2 heures à la température de reflux du milieu réactionnel, à raison d'au moins 2 moles de $CH_3COCl$ (de préférence 3 à 5 moles de $CH_3COCl$) pour 1 mole de V.

D'une manière générale on préfère mettre en oeuvre la variante A plutôt que la variante B pour la synthèse de l'ensemble des dérivés de 1 - (acétylaminophényl) - 2 - amino - propanone. La variante B n'intervient en

pratique que pour les composés Y=Z=halogène.

Les présents dérivés de 1 - (acétylaminophényl) - 2-amino - propanone sont utiles en thérapeutique. Ils agissent tous sur le SNC, plus précisément en tant qu'agents antidépresseurs. Ils présentent dans leur profil neuropsychopharmacologique une composante stimulante ou excitante. Certains de ces dérivés présentent en outre, à côté de ces effets sur le SNC, des propriétés immunologiques intéressantes; en particulier les produits des exemples 7 (CRL 41 220) 12 (CRL 41 242) 14 (CRL 41 247), 15 (CRL 41 254) et 17 (CRL 41 262) ont des effets immuno-modulateurs bénéfiques dans le traitement des patients ayant une réaction immunitaire insuffisante, les produits les plus intéressants eu égard à leurs propriétés immunologiques étant les produits des exemples 7 (CRL 41 220), 12 (CRL 41 242) et 17 (CRL 41 262).

Selon l'invention on préconise une composition thérapeutique qui est caractérisée en ce qu'elle renferme, en association avec un excipient physiologiquement acceptable, au moins un dérivé de 1 - (acétylaminophényl) - 2 - amino - propanone ou l'un de ses sels d'addition non-toxiques, en tant que principe actif.

Bien entendu, dans une telle composition le principe actif intervient en quantité pharmaceutiquement efficace.

D'autres avantages et caractéristiques de l'invention seront mieux compris à la lecture qui va suivre, d'une part, d'exemple de préparation, et, d'autre part, de résultats d'essais pharmacologiques, l'ensemble de ces éléments n'étant nullement limitatif mais donné à titre d'illustration.

**PREPARATION I**

Obtention du chlorhydrate de 1 - (4 - acétylamino - 3,5-dichlorophényl) - 2 - isopropylamino - propanone.

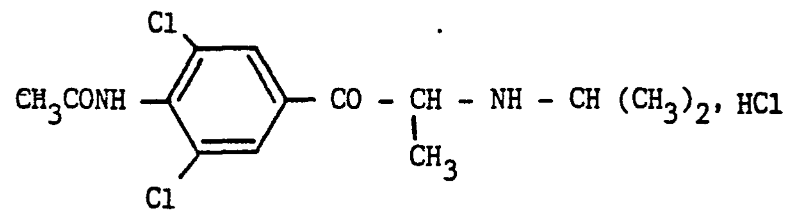

(Exemple 7; N° de Code: CRL 41 220)

On dissout 28 g de dichlorhydrate de 1 - (4-aminophényl)-2 - isopropylamino - propanone dans 100 ml d'eau distillée. Dans cette solution maintenue à 10 ° C on ajoute 0,2 mole de $Cl_2$ par barbotage. On agite pendant 2 heures puis évapore à sec sous vide.

Le résidu d'évaporation, qui est solide et de couleur brune, est repris dans l'acide acétique et donne par recristallisation 11 g (rendement: 35 %) de dichlorhydrate de 1 - (4 - amino - 3,5-dichlorophényl) - 2 - isopropylamino - propanone.

$F_{inst.} > 260°C$.

On porte au reflux pendant 4 heures un mélange des 11 g du produit ainsi obtenu avec 50 ml de $CH_3COCl$. On évapore ensuite l'excès de chlorure d'acétyle sous vide. Le résidu d'évaporation est repris par de l'éthanol pour donner 9 g (rendement: 25 %) de CRL 41 220.

$F_{inst.} > 260°C$.

**PREPARATION II:**

Obtention du chlorhydrate de 1 - (4 - acétylaminophényl) - 2-éthylamino - propanone.

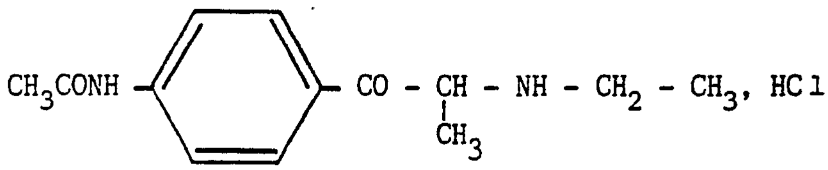

(Exemple 8, N° de Code: CRL 41 224)

a/ 1 - (4 - Acétylaminophényl) - 2 - chloro - propanone.

On coule en 40 minutes 92 g (0,72 mole) de chlorure de 2 - chloropropionyle [-Cl-CO-CHCl-$CH_3$] dans une suspension constituée par 54 g (0,40 mole) d'acétanilide, 160 g (1,20 mole) de chlorure d'aluminium et 400 ml de sulfure de carbone. On chauffe pendant 1 heure au reflux puis verse le milieu réactionnel sur un mélange eau - glace acidulé avec de l'acide HCl dilué, et agite ensuite pendant une nuit à la température ambiante (15 -25°C). Le précipité formé est recueilli par filtration puis purifié par recristallisation dans le benzène pour donner 78 g (rendement: 86,5 %) de 1 - (4 - acétylaminophényl) 2 - chloro - propanone.

$F_{inst.}$ = 120 °C.

b/ CRL 41 224.

On chauffe vers 60 - 70 °C pendant 1 heure une solution de 25 g (0,11 mole) de 1 - (4-acétylaminophényl) - 2 - chloropropanone dans 164 ml (1,10 mole) d'éthylamine en solution aqueuse à 330 g/l. On évapore l'excès d'éthylamine sous pression réduite, extrait le résidu d'évaporation par plusieurs fractions d'acétate d'éthyle, puis traite la phase acétate d'éthyle séchée au moyen d'éthanol chlorhydrique. Le précipité formé est purifié par lavage à chaud dans de l'éthanol anhydre pour donner 13,3 g (rendement du stade b: 44,7 %; rendement global: 38,7 %) de CRL 41 224.

$F_{inst.}$ = 230°C (avec décomposition).

**PREPARATION III.**

Obtention du chlorhydrate de 1 - (4 - acétylaminophényl) - 2-pipéridino - propanone.

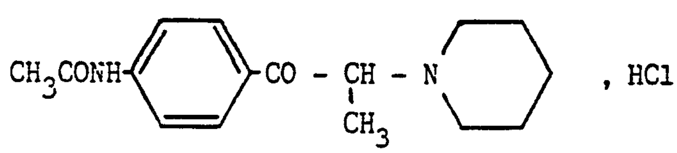

(Exemple 11; N° de Code: CRL 41 240)

On agite pendant 1 heure à la température ambiante (15-25°C) puis pendant 0,5 heure au reflux une solution constituée par 18 g (0,0798 mole) de 1 - (4 - acétylaminophényl) - 2 - chloropropanone, 78,8 ml (0,7980 mole)de pipéridine et 50 ml d'eau. On évapore sous pression réduite jusqu'à siccité, on reprend le résidu d'évaporation avec de l'acétate d'éthyle. On lave la phase acétate d'éthyle avec de l'eau, séche sur $Na_2SO_4$ et évapore le solvant pour donner 25 g d'une huile de couleur brun - orangé. Cette huile est traitée dans de l'acétate d'éthyle par de l'éthanol chlorhydrique.

Après purification par cristallisation et traitement au noir de carbone (noir CXA) dans l'éthanol anhydre, on

obtient 19,8 g (rendement: 79,9 %) de CRL 41 240. $F_{inst.}$ = 240° C (avec décomposition).

**PREPARATION IV**

Obtention du dichlorhydrate de 1 - (4 - acétylaminophényl) - 2-(4 - méthylpipérazino) - propanone.

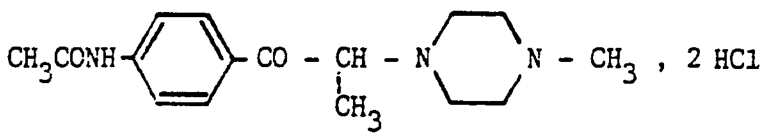

(Exemple 12; N° de Code: CRL 41 242)

On agite pendant 1 heure à la température ambiante puis pendant 0,5 heure au reflux un mélange de 18,5 g (0,082 mole) de 1 - (4 - acétylaminophényl) - 2 - chloro - propanone et de 91 ml (0,820 mole) de 4 - méthylpipérazine dans 60 ml $H_2O$. On reprend le milieu réactionnel avec 100 ml $H_2O$ et isole par filtration 11,6 g de 1 - (4 - acétylaminophényl) - 2 - (4 - méthylpipérazino)- propanone ($F_{inst.}$. = 90° C).

La base libre ainsi obtenue est dissoute dans l'éthanol anhydre, traitée au noir de carbone (noir CXA) puis avec de l'éthanol chlorhydrique. Le précipité formé est isolé par filtration pour donner 14,1 g (rendement: 47,5 %) de CRL 41 242. $F_{inst.}$ =230° C (avec décomposition).

**PREPARATION V**

Obtention du chlorhydrate de 1 - (4-acétylamino-3-chlorophényl)-2-pyrrolidino - propanone.

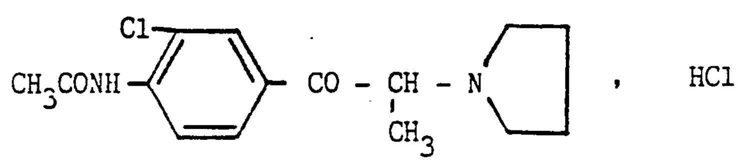

(Exemple 6; N° de Code: CRL 41 219)

1a/ 1 - (4 - Acétylamino - 3 - chlorophényl) - 2 - chloro- propanone.

On met en suspension 45 g (0,199 mole) de 1 - (4-acétylaminophényl) - 2 - chloro - propanone dans 400 ml de $CHCl_3$. On introduit 0,2 mole de $Cl_2$ par barbotage. On obtient une solution jaune que l'on évapore à sec. Par recristallisation dans le toluène du résidu d'évaporation on obtient 26 g (rendement: 50 %) de 1 - (4 - acétylamino - 3 - chlorophényl) - 2 - chloro- propanone.

$F_{inst.}$ = 118° C.

b/ CRL 41 219

On dissout 26 g de 1 - (4 - acétylamino - 3 - chlorophényl) - 2 - chloro - propanone dans un mélange de 100 ml de pyrrolidine et 20 ml d'eau. On porte au reflux pendant 2 heures, évapore sous vide la pyrrolidine en excès. Le résidu d'évaporation, qui est huileux, est repris dans $C_2H_5OH$ et précipité au moyen de HCl gazeux. On obtient 8 g (rendement: 24 %) de CRL 41 219.

$F_{inst.}$ = 260 °C.

**PREPARATION VI**

Obtention du chlorhydrate de 1 - (3 - acétylaminophényl) - 2- pipéridino - propanone.

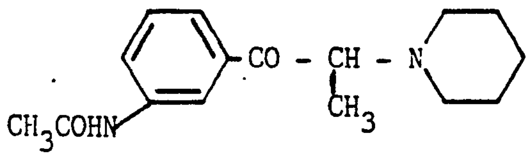

, HCl

(Exemple 17; N° de Code: CRL 41 262)

a/ 1 - (3 - Acétylaminophényl) - 2 - bromo - propanone.

Dans une solution de 37 g (0,19 mole) de 1 - (3-acétylaminophényl) - propanone dans un mélange de 300 ml de tétrahydrofuranne et de 300 ml d'éther diéthylique, et en présence d'une trace de $AlCl_3$ on coule à la température ambiante en 2,5 heures 30,4 g (0,19 mole) de brome. On dégaze le milieu réactionnel vers 40°C au moyen d'un courant d'azote et évapore à sec sous pression réduite. Le résidu d'évaporation est purifié par cristallisation dans l'acétate d'éthyle pour donner 26,5 g (rendement: 51,6 %) de 1 - (3 - acétylaminophényl) - 2 - bromo - propanone.

$F_{inst.}$ = 133°C.

b/ CRL 41 262.

On agite pendant 1 heure à la température ambiante un mélange de 18,5 g (0,0685 mole) de 1 - (3 - acétylaminophényl)-2 - bromo - propanone, de 68 ml (0,685 mole) de pipéridine et de 45 ml d'eau. On amène le milieu réactionnel à siccité sous pression réduite, reprend le résidu d'évaporation par de l'acétate d'éthyle et élimine l'insoluble par filtration. Le filtrat est lavé à l'eau, séché sur $Na_2SO_4$ puis traité avec de l'éthanol chlorhydrique. Le précipité formé est purifié par recristallisation dans le mélange $C_2H_5OH$ - $CH_3OH$ (3:5) v/v pour donner 15 g (rendement: 70,7%) de CRL 41 262. $F_{inst.}$ = 245°C (avec décomposition).

**PREPARATION VII**

Obtention du chlorhydrate de 1-(4-acétylaminophényl)-2-isopropylamino-propanone.

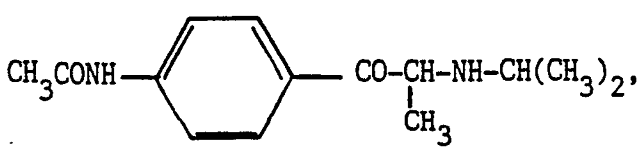

, HCl

(Exemple 1; N° de Code: CRL 41 152).

a) 1-(4-Acétylaminophényl)-2-chloro-propanone

On introduit 27 g d'acétanilide dans un mélange comprenant 67 g de $AlCl_3$ et 76,8 g de chlorure de 2-chloropropionyle [$Cl-CO-CHCl-CH_3$], à une température comprise entre 35 et 45°C. On agite jusqu'à dissolution

totale de l'acétanilide. Le milieu réactionnel ainsi obtenu est versé sur de la glace pilée; le précipité formé est recueilli par filtration, lavé à l'eau jusqu'à pH7, et séché pour donner 39 g (rendement: 86%) de 1-(4-acétylaminophényl)-2-chloro-propanone. $F_{inst.}$ = 124° C.

b) CRL 41 152

Les 39 g de 1-(4-acétylaminophényl)-2-chloro-propanone ainsi obtenus sont dissous dans 200 ml d'isopropylamine. Le milieu réactionnel résultant est chauffé au reflux pendant 6 heures. On évapore sous vide l'isopropylamine en excès. Le résidu d'évaporation qui se présente sous la forme d'une huile visqueuse est repris par 200 ml d'éthanol anhydre et on précipite le chlorhydrate au moyen d'un courant de HCl gazeux. Après recristallisation dans l'éthanol, on obtient 40 g (rendement: 70%) de CRL 41 152 qui se présente sous la forme d'une poudre blanche très soluble dans l'eau. F = 240° C.

## PREPARATION VIII

Obtention du chlorhydrate de 1-(4-acétylamino-3- chloro-phényl)-2-isopropylamino- propanone.
(Exemple 3; N° de Code CRL 41 192).

a) 1-(4-Acétylamino-3-chlorophényl)-2-chloro-propanone

45 g de 1-(4-acétylaminophényl)-2-chloro-propanone sont mis en suspension dans 400 ml de $CHCl_3$. On introduit 0,2 mole de $Cl_2$ par barbotage. La solution jaune résultante est évaporée à sec.

Par recristallisation du résidu d'évaporation au moyen de toluène on obtient 26 g (rendement: 50%) de 1-(4-acétylamino-3-chlorophényl)-2-chloro-propanone.

$F_{inst.}$ = 118° C.

b) CRL 41 192

On dissout les 26 g de 1-(4-acétylamino-3-chlorophényl)-2-chloro-propanone, ainsi obtenus, dans 150 ml d'isopropylamine. On porte au reflux pendant 4 heures, évapore sous vide l'isopropylamine en excès. Le résidu d'évaporation qui se présente sous la forme d'une huile est repris dans l'éthanol, et on précipite le chlorhydrate au moyen d'un courant gazeux d'HCl.

On obtient 9,6 g (rendement: 15 %) de CRL 41 192. F = 260° C.

On a résumé ci-après les résultats des essais qui ont été entrepris avec les composés selon l'invention.

## A. ESSAIS RELATIFS AU CRL 41 224 (PRODUIT DE L'EXEMPLE 8).

Dans l'étude neuropsychopharmacologique qui suit, le CRL 41 224 en solution dans de l'eau distillée (pH6) a été administré par voie intrapéritonéale sous un volume de 20 ml/kg chez la souris mâle et de 5 ml/kg chez le rat mâle.

## I. TOXICITE

Chez la souris mâle la DL-O (dose maximale non mortelle) par voie intrapéritonéale est supérieure à 256 mg/kg et inférieure à 512 mg/kg.

## II. COMPORTEMENT GLOBAL ET REACTIVITES

Des lots de trois animaux sont observés avant, puis 0,25 h, 0,50 h, 1 h, 2 h, 3 h, et 24 h après l'administration de CRL 41 224. On constate:

1°/ chez la souris

- aux doses de 2 et 8 mg/kg

des comportements, réactivités, variations du diamètre pupillaire et de la température rectale, sensiblement comparables à ceux du lot témoin ne recevant que de l'eau distillée;

- à la dose de 32 mg/kg,
- une excitation pendant 3 h,
- des stéréotypies durant entre 2 et 3 h,
- une augmentation de la réactivité au toucher et du tonus musculaire entre 2 et 3 h;
- à la dose de 128 mg/kg,
- une excitation pendant 3 h,
- des stéréotypies durant entre 2 et 3 h,
- une augmentation de la réactivité au toucher et du tonus musculaire pendant 24 h,
- une hyperthermie entre 2 et 3 h (+ 0,9 ° C), et

- une mydriase modérée pendant 3 h;

2°/ chez le rat,

- à la dose de 1 mg/kg,
- une mydriase brève se manifestant 0,5 h après administration du CRL 41 224;
- à la dose de 4 mg/kg,
- une mydriase durant 2 h;
- à la dose de 16 mg/kg,
- des stéréotypies pendant 3 h,
- une mydriase durant 3 h,
- à la dose de 64 mg/kg,
- une excitation pendant 1 h,
- des stéréotypies pendant 3 h,
- une piloérection pendant 1 h,
- une augmentation de la réactivité au toucher et du tonus musculaire pendant 3 h,
- une hyperthermie pendant 3 h (+1,5°C),

et

- une mydriase pendant 3 h.

## III. INTERACTION AVEC L'APOMORPHINE

1/ chez la souris

Des lots de 6 souris reçoivent le CRL 41 224 0,5 h avant l'injection sous-cutanée de 1 ou 16 mg/kg d'apomorphine. On observe que aux doses de 8 mg/kg, 32 mg/kg et 128 mg/kg, le CRL 41 224 s'oppose à l'hypothermie induite par l'apomorphine sans modifier les comportements de verticalisation et de stéréotypies.

2/ chez le rat

Le CRL 41 224 est administré à des lots de 6 rats 0,5 h avant l'injection sous-cutanée de 0,5 mg/kg d'apomorphine. On observe que, à la dose de 16 mg/kg, mais surtout à la dose de 64 mg/kg, le CRL 41 224 entraîne une potentialisation des stéréotypies induites par l'apomorphine.

## IV. INTERACTION AVEC L'AMPHETAMINE

L'amphétamine (2mg/kg) est injectée par voie intrapéritonéale à des lots de 6 rats, 30 minutes après l'administration CRL 41 224. On constate que, à la dose de 16 mg/kg, mais surtout à la dose de 64 mg/kg, le CRL 41 224 entraîne une potentialisation des stéréotypies amphétaminiques.

## V. INTERACTION AVEC LA RESERPINE

Quatre heures après l'injection intrapéritonéale de 2,5 mg/kg de réserpine, des lots de 6 souris reçoivent le CRL 41 224.

On note que dès la dose de 2 mg/kg, le CRL 41 224 s'oppose à l'hypothermie induite par la réserpine. Cet antagonisme est important pour les doses de 32 mg/kg et 128 mg/kg, et, à la dose de 128 mg/kg, le CRL 41 224 diminue l'intensité du ptôsis réserpinique.

## VI. INTERACTION AVEC L'OXOTREMORINE

Le CRL 41 224 est administré à des lots de 6 souris 0,5 h avant l'injection intrapéritonéale de 0,5 mg/kg d'oxotrémorine.

1/ Action sur la température

On constate que, aux doses de 2 mg/kg, 8 mg/kg, mais surtout aux doses de 32 mg/kg et 128 mg/kg, le CRL 41 224 s'oppose à l'action hypothermisante de l'oxotrémorine.

2/ Action sur les tremblements

On constate que, aux doses de 8 mg/kg, 32 mg/kg, mais surtout 128 mg/kg, le CRL 41 224 diminue nettement l'intensité des tremblements induits par l'oxotrémorine.

3/ Action sur les symptômes cholinergiques périphériques

On observe que le CRL 41 224 ne modifie pratiquement pas les signes de stimulation cholinergique périphérique induits par l'oxotrémorine.

## VII. ACTION SUR LE TEST DES QUATRE PLAQUES, LA TRACTION ET L'ELECTROCHOC.

Le test est pratiqué sur des lots de 10 souris, 30 minutes après l'administration de CRL 41 224.

On constate que, à la dose de 128 mg/kg, le CRL 41 224 entraîne une augmentation du nombre de passages punis, ne provoque pas d'incapacité motrice majeure, n'aggrave pas les effets convulsivants et ne modifie pas les effets létaux de l'électrochoc.

## VIII. ACTION SUR LA MOTILITE SPONTANEE

0,5 h après avoir reçu le CRL 41 224, les souris (6 par dose, 12 témoins) sont placées en actimètre où leur motilité est enregistrée pendant 30 minutes.

On observe que, à la dose de 8 mg/kg et surtout aux doses de 32 mg/kg et 128 mg/kg, le CRL 41 224 augmente l'activité motrice spontanée de la souris.

## IX.ACTION SUR L'AGRESSIVITE INTERGROUPES

Après avoir séjourné pendant 3 semaines dans chacune des moitiés d'une cage séparées par une cloison opaque, des groupes de 3 souris reçoivent le CRL 41 224. Une demi-heure plus tard, les deux groupes d'une même cage sont réunis par retrait de la cloison et on note le nombre de combats qui surviennent en 10 minutes. Le test est pratiqué pour moitié sur les souris habituelles (NMRI, C.E.R. Janvier) et pour moitié sur des souris NMRI (Iffa Credo).

On constate que, à la dose de 128 mg/kg, le CRL 41 224 diminue le nombre de combats.

## X. ACTION VIS-A-VIS DE QUELQUES COMPORTEMENTS PERTURBES PAR DIVERS AGENTS.

1/ Motilité réduite par habituation à l'enceinte

Après 18 heures de séjour dans les actimètres, les souris (6 par dose, 12 témoins) reçoivent le CRL 41 224. Elles sont aussitôt replacées dans leurs enceintes respectives et, une demi-heure plus tard, on enregistre leur motilité pendant 30 minutes.

On observe que, aux doses de 32 mg/kg et de 128 mg/kg, le CRL 41 224 entraîne une reprise nette de l'activité motrice chez la souris habituée à son enceinte.

2/ Motilité réduite par agression hypoxique.

Une demi-heure après avoir reçu le CRL 41 224, les souris (10 par dose, 20 témoins) sont soumises à une anoxie hypobare aigue [dépression de 600 mmHg (i.e. environ 8 x $10^4$ Pa) en 90 secondes; détente de 45 secondes 7, puis elles sont placées en actimètre où leur motilité est enregistrée pendant 10 minutes.

On observe que,aux doses de 8 mg/kg, 32 mg/kg, et surtout 128 mg/kg, le CRL 41 224 entraîne une amélioration nette de la récupération motrice chez la souris dont la motilité a été déprimée à la suite d'un séjour bref dans une enceinte à pression réduite.

3/ Anoxie asphyxique

Des lots de 10 souris reçoivent le CRL 41 224 une demi-heure avant l'administration intrapéritonéale de 32 mg/kg de triiodoéthylate de gallamine (agent curarisant de référence).

On observe que le CRL 41 224 ne modifie pas le délai d'apparition des convulsions et de la mort consécutives à une anoxie asphyxique provoquée par un curarisant.

## XI. INTERACTION AVEC LE BARBITAL

Une demi-heure après l'administration de CRL 41 224, des lots de 10 souris reçoivent une injection intrapéritonéale de barbital (220 mg/kg).

On constate que, dès la dose de 2 mg/kg, le CRL 41 224 diminue la durée du sommeil barbiturique (l'antagonisme est total pour 32 mg/kg).

**XII. ACTION SUR LE "DESESPOIR COMPORTEMENTAL"**

Une demi-heure après avoir reçu le CRL 41 224, des lots de 6 souris sont placés dans un bécher rempli d'eau sur une hauteur de 6 cm. On note la durée totale d'immobilité entre la 2ème et la 6ème minutes suivant l'immersion.

On observe que, aux doses de 8 mg/kg et surtout à 32 mg/kg et 128 mg/kg, le CRL 41 224 diminue la durée de l'immobilité de la souris placée en immersion forcée.

**XIII. CONCLUSIONS**

Il résulte de l'ensemble des essais neuropsychopharmacologique ci-dessus que le CRL 41 224 présente des effets

- antidépresseurs: antagonisme des hypothermies induites par l'apomorphine, la réserpine ou l'oxotrémorine, et, diminution de la durée de l'immobilité de "désespoir";

- stimulants: excitation chez la souris, présence de mouvements stéréotypés chez la souris et chez le rat, potentialisation des stéréotypies induites par l'apomorphine et l'amphétamine, augmentation de l'activité motrice, amélioration de la récupération motrice chez la souris dont la motilité a été déprimée à la suite d'un séjour bref dans une enceinte à pression réduite, et, reprise nette de l'activité motrice chez la souris habituée à son enceinte; et,

- stimulants α -adrénergiques périphériques: mydriase et antagonisme du ptôsis réserpinique, piloérection.

Il s'ensuit que le CRL 41 224 se comporte comme un agent antidépresseur du SNC. L'effet antidépresseur est associé, aux mêmes doses, à une composante stimulante nette.

**B. ESSAIS RELATIFS AU CRL 41 219 (PRODUIT DE L'EXEMPLE 6)**

L'étude neuropsychopharmacologique du CRL 41 219 a été réalisée comme indiqué ci-dessus pour le CRL 41 224. Les résultats ont été donnés ci-après.

Chez la souris mâle, par voie intrapéritonéale, la DL-30 (dose mortelle pour 30 % des animaux testés) est de l'ordre de 128 mg/kg, et la DL-50 est de l'ordre de 200 mg/kg environ.

En bref, le CRL 41 219 présente un profil caractérisé par

- des effets stimulants
- chez la souris:
- excitation avec hyper-réactivité,
- hyperactivité(augmentation de l'activité motrice spontanée, reprise de l'activité motrice après habituation à l'enceinte, amélioration de la récupération motrice après agression hypoxique, et augmentation du nombre de passages punis au test des 4 plaques),
- présence tardive de stereotypies,
- antagonisme du sommeil barbiturique;
- chez le rat:
- excitation (avec hyper-réactivité),
- présence de mouvements stéréotypés et potentialisation des stéréotypies induites par l'apomorphine et l'amphétamine;
- des effets antidépresseurs:
- antagonismes des hypothermies induites par l'apomorphine la réserpine et l'oxotrémorine,
- diminution de l'immobilité dite de "désespoir";
- des effets reflétant une stimulation α- adrénergique périphérique:
- antagonisme du ptôsis réserpine,
- mydriase,
- antagonisme des tremblements provoques par l'oxotrémorine.

De plus le CRL 41 219 semble favoriser ou potentialiser les effets des moyens convulsivants (aggravation des effets létaux de électrochoc, diminution des délais d'apparition des convulsions et de la mortalité consécutives à une anoxie asphyxique).

Il résulte de l'ensemble de ces résultats que le CRL 41 219 est une substance douée de propriétés antidépressives, stimulantes et éveillantes.

**C. ESSAIS RELATIFS AU CRL 41 220 (PRODUIT DE L'EXEMPLE 7)**

L'étude neuropsychopharmacologique du CRL 41 220 a été réalisée selon les modalités opératoires données ci-dessus pour le CRL 41 224.

## I. TOXICITE

Par voie intrapéritonéale chez la souris mâle, le CRL 41 220 présente les DL-0, DL-30 et DL-50 suivantes:
DL-0: supérieure à 128 mg/kg,
DL-30: de l'ordre de 250 mg/kg environ,
DL-50; de l'ordre de 500 mg/kg environ.

## II. ACTION SUR LE SNC

Le CRL 41 220 présente des effets antidépresseurs et stimulants.

## III. ACTION CARDIOVASCULAIRE

Quatre chiens (poids moyen: 13,8 kg) anesthésiés au nembutal reçoivent, par voie intraduodénale, le CRL 41 220 aux doses successives de 0,1 mg/kg, 0,5 mg/kg, 1 mg/kg, 2,5 mg/kg, 5 mg/kg et 10 mg/kg. On mesure la pression artérielle, la fréquence cardiaque, le débit artériel de l'artère fémorale, le débit artériel de l'artère vertébrale, la température rectale et/ou cutanée, et, on observe la coloration de la peau et de la bile (recueillie par cathétérisation du cholédoque après ligature du cystique).

On constate que le CRL 41 220, administré par voie intraduodénale

- augmente le débit vertébral dès la dose de 0,5 mg/kg, le débit fémoral à la dose de 1 mg/kg, et la fréquence cardiaque à la dose de 2,5 mg/kg,
- présente un effet hypotenseur à la dose de 10 mg/kg,
- induit une stimulation respiratoire chez les 4 chiens,
- augmente les températures rectale et cutanée.

Les effets de l'isoprénaline, testés après administration introduodénale à la dose cumulée de 19,1 mg/kg de CRL 41 220 ne sont pas modifiés en ce qui concerne la fréquence cardiaque, et sont très légèrement modifiés en ce qui concerne la pression artérielle diastolique: avec 10 µg/kg d'isoprénaline la pression artérielle diastolique passe de 112 mm Hg (i.e. environ 1,49 x $10^4$ Pa) à 47 mmHg (i.e. environ 6,26 x $10^3$ Pa) au lieu de 158 mmHg (i.e. environ 2,1 x $10^4$ Pa) en témoin, et, la fréquence cardiaque passe de 221 battements/min. à 275 battements/min. au lieu de 175 battements/min. à 280 battements/min. en témoin (les chiens étant utilisés en contrôle comme témoins par rapport à eux-mêmes).

L'hypertension induite par la noradrénaline est diminuée: avec 2 µg/kg de noradrénaline la pression artérielle systolique passe de 202 mmHg (i.e. environ 2,69 x $10^4$ Pa) à 272 mmHg (i.e. environ 3,62 x $10^4$ Pa) après administration intraduodénale de CRL 41 220, au lieu de 199 mmHg (i.e. environ 2,65 x $10^4$ Pa) a 323 mmHg (i.e. environ 4,3 x $10^4$ Pa) en témoin.

En bref le CRL 41 220 agit en tant que hypotenseur (les effets hypotenseurs résultant de la diminution de la pression artérielle diastolique). Comme l'injection de 1 mg/kg de propanolol en fin d'essai supprime tous les effets du CRL 41 220, on présume que ce produit agit sur le système cardiovasculaire en stimulant les récepteurs β - adrénergiques.

## IV. PROPRIETES IMMUNOLOGIQUES

Le test des cellules formant des plages de lyses selon la technique de A.J. CUNNINGHAM et al. ("Further improvements in the plaque technique for detecting single antibody forming cell's), Immmunology 14, pages 599-601 (1968), et la mesure de l'intensité de l'hypersensibilité retardée aux globules rouges de mouton selon la technique de T.E. MILLER et al. ("Immunopotentiation with BCG II modulation of the response to sheep blood cells"), Journal of the National Cancer Institute 51, (N° 5), pages 1669-1676 (1973), ont permis de mettre en évidence le pouvoir stimulant immuno-modulateur du CRL 41 220.

## V ESSAIS COMPLEMENTAIRES

Le CRL 41 220 s'est avéré actif dans l'étude du carcinome de LEWIS chez la souris, selon le protocole suivant:

a/ animaux: souris femelles consanguines ($C_{57}BL_6$),
b/ cellules infectantes: 10 cellules injectées en sous-cutané sur le dos des animaux,
c/ surveillance de la croissance de la tumeur par mesure de celle-ci deux fois par semaine,
d/ évaluation des métastases pulmonaires après fixation des poumons des animaux morts dans le liquide de

BOUIN.

Dans ces conditions opératoires, le CRL 41 220 utilisé seul paraît inactif, alors que le cyclophosphamide (agent anticancéreux de référence), à la dose de 100 mg/kg, diminue le nombre de métastases pulmonaires et ralentit faiblement le développement des tumeurs sans améliorer cependant la survie des animaux traités, d'une part, et, à la dose de 150 mg/kg, s'oppose nettement au processus déclenché par l'administration des cellules infectantes (survie de 2 animaux sur 6), d'autre part.

Selon les mêmes modalités opératoires l'association du CRL 41 220 avec 150 mg/kg de cyclophosphamide donne les résultats suivants consignés dans le tableau II.

**TABLEAU II**

| association | | résultats |
|---|---|---|
| cyclophosphamide | CRL 41 220 | |
| 150 mg/kg | 100 mg/kg | augmentationde la toxicité par rapport au cyclophosphamide utilisé seul |
| 150 mg/kg | 5 mg/kg | sensiblement peu de différences par rapport au cyclophosphamide utilisé seul |
| 150 mg/kg | 2 mg/kg | action protectrice indiscutable vis à vis du carcinome de LEWIS, tous les animaux traités étant encore en vie plusieurs semaines après l'expérimentation. |

**VI. ESSAIS CLINIQUES**

Chez l'homme, par voie orale, à la dose de 250 à 500 mg (par gélule ou comprimé), le CRL 41 220 a donné de bons résultats dans le traitement des patients qui présentaient des troubles circulatoires et dont la réaction immunitaire était insuffisante, par exemple dans les cas d'herpès récidivants, de polyarthrites, rhumatoïdes et de rougeoles à forme sévère.

**D. ESSAIS RELATIFS AU CRL 41 232 (PRODUIT DE L'EXEMPLE 9)**

L'étude neuropsychopharmacologique du CRL 41 232 a été réalisée selon les modalités opératoires données ci-dessus pour le CRL 41 224.

## I. TOXICITE

Chez la souris mâle par voie intrapéritonéale, le CRL 41 232 présente
- une DL-0 supérieure à 128 mg/kg, et
- une DL-30 de l'ordre de 250 mg/kg environ.

## II. ACTION SUR LE SNC

En bref le CRL 41 232 présente
- des effets antidépresseurs: antagonisme des hypothermies induites par l'apomorphine, la réserpine et l'oxotrémorine, et, diminution de la durée de l'immobilité de "désespoir";
- des effets stimulants: excitation chez la souris, présence de mouvements stéréotypés chez la souris et le rat, potentialisation des stéréotypies induites par l'apomorphine et l'amphétamine, augmentation de l'activité motrice, amélioration de la récupération motrice chez la souris dont la motilité a été déprimée à la suite d'un séjour bref dans une enceinte à pression réduite, et reprise nette de l'activité motrice chez la souris habituée à son enceinte; et,
- une stimulation α -adrénergique périphérique: mydriase, exophtalmie, et, antagonisme du ptôsis réserpinique.

Le CRL 41 232 se comporte dans l'ensemble comme un agent stimulant et antidépresseur du SNC.

# E. ESSAIS RELATIFS AU CRL 41 236 (PRODUIT DE L'EXEMPLE 10)

L'étude neuropsychopharmacologique du CRL 41 236 a été réalisée selon les modalités opératoires données ci-dessus pour le CRL 41 224.

## I. TOXICITE

Par voie intrapéritonéale chez la souris mâle, le CRL 41 236 présente
- une DL-0 supérieure à 256 mg/kg, et
- une DL-30 de l'ordre de 500 mg/kg environ.

## II. ACTION SUR LE SNC

En bref le CRL 41 236 possède
- des effets antidépresseurs: antagonisme des hypothermies induites par l'apomorphine, la réserpine et l'oxotrémorine, et, diminution de l'immobilité de "désespoir";
- des effets stimulants: excitation avec hyperréactivité sans la présence de mouvements stéréotypés, hyperactivité, augmentation de la motilité spontanée,de l'activité motrice après habituation à l'enceinte, amélioration de la récupération motrice après agression hypoxique, et augmentation modérée du nombre de passages punis au test des 4 plaques, potentialisation des stéréotypies induites par l'apomorphine et l'amphétamine et, antagonisme du sommeil barbiturique;
- une stimulation α -adrénergique périphérique: antagonisme du ptôsis réserpinique, et mydriase.

De plus, le CRL 41 236 semble aggraver les effets létaux de l'électrochoc.

Il en résulte que le CRL 41 236 se comporte comme un agent antidépresseur ayant des propriétés stimulantes et éveillantes.

# F. ESSAIS RELATIFS AU CRL 41 240 (PRODUIT DE L'EXEMPLE 11)

Selon les modalités opératoires décrites ci-dessus pour l'étude du CRL 41 224, les propriétés suivantes ont été observées.

**I. TOXICITE**

Par voie intrapéritonéale, chez la souris mâle, le CRL 41 240 présente une DL-0 supérieure à 256 mg/kg.

**II. ACTION SUR LE SNC**

En bref le CRL 41 240 possède
- des effets antidépresseurs: antagonisme des hypothermies induites par l'apomorphine, la réserpine et l'oxotrémorine, et, diminution de la durée d'immobilité de "désespoir" (cette diminution étant peut être en relation avec la composante stimulante);
- des effets stimulants et éveillants: excitation chez la souris et chez le rat avec présence de mouvements stéréotypés et potentialisation des stéréotypies induites par l'apomorphine et l'amphétamine, augmentation de l'activité motrice, amélioration de la récupération motrice chez la souris dont la motilité a été déprimée à la suite d'un séjour bref dans une enceinte à pression réduite, reprise de l'activité chez la souris habituée à son enceinte, et, antagonisme très net du sommeil barbiturique;
- une stimulation α-adrénergique périphérique: mydriase, exophtalmie, et antagonisme du ptôsis réserpinique; et
- des effets anticonvulsivants; à dose élevée: antagonisme des effets convulsivants de l'électrochoc, et, augmentation du délai d'apparition des convulsions asphyxiques.

Il s'ensuit que le CRL 41 240 se comporte comme un agent antidépresseur et stimulant du SNC.

**G. ESSAIS RELATIFS AU CRL 41 242 (PRODUIT DE L'EXEMPLE 12)**

L'étude neuropsychopharmacologique du CRL 41 242 a été effectuée selon les modalités opératoires décrites ci-dessus pour le CRL 41 224, avec la différence que le pH de la solution aqueuse de CRL 41 242 à injecter par voie intrapéritonéale varie en fonction de la concentration comme indiqué dans le tableau III ci-après.

**TABLEAU III**

pH DE LA SOLUTION AQUEUSE A INJECTER EN FONCTION DE LA CONCENTRATION EN CRL 41 242.

| Concentration en CRL 41 242 | pH |
|---|---|
| 50 g/l | 2,5 |
| 13 g/l | 3,0 |
| 2 g/l | 3,5 |
| 0,8 g/l | 4,0 |
| 0,4 g/l | 4,5 |
| 0,2 g/l | 5,0 |
| ≤ 0,05 g/l | 5,5 |

**I. TOXICITE**

Par voie intrapéritonéale chez la souris mâle, le CRL 41 242 présente une DL-0 supérieure à 256 mg/kg et une DL-60 de l'ordre de 500 mg/kg environ.

**II. ACTION SUR LE SNC**

En bref le CRL 41 242 présente à doses élevées
- des effets sédatifs objectivés par:
- une sédation chez la souris et le rat avec diminution des réactivités et hypothermie chez la souris,
- une diminution de la motilité spontanée et du nombre de passages punis au test des quatre plaques chez la souris,
- une diminution de l'agressivité intergroupes chez la souris,
- une augmentation de la durée du sommeil barbiturique chez la souris,
- une augmentation du temps d'apparition des convulsions et de la mort consécutives à une anoxie asphyxique; et
- des effets antidépresseurs objectivés par
- un antagonisme très modéré de l'hypothermie induite par l'oxotrémorine, et
- une faible diminution de la durée d'immobilité de la souris placée en immersion forcée.

Le CRL 41 242 présente par ailleurs aux faibles doses des effets de type stimulant très discrets.

**III. PROPRIETES IMMUNOLOGIQUES**

Le CRL 41 242 s'est révélé être particulièrement intéressant en tant que substance immunostimulante.

**H. ESSAIS RELATIFS AU CRL 41 245 (PRODUIT DE L'EXEMPLE 13)**

L'étude neuropsychopharmacologique du CRL 41 245 a été effectuée selon les modalités décrites ci-dessus pour le CRL 41 224.

**I. TOXICITE**

Par voie intrapéritonéale chez la souris mâle, le CRL 41 245 présente une DL-0 supérieure à 256 mg/kg et une DL-100 inférieure ou égale à 512 mg/kg environ.

## II. ACTION SUR LE SNC

En bref dans les conditions expérimentales, le CRL 41 245 présente

- des effets antidépresseurs:
- antagonisme des hypothermies induites par l'apomorphine, la réserpine et l'oxotrémorine,
- diminution de la durée d'immobilité de la souris placée en immersion forcée (peut-être liée à une composante stimulante);
- des effets stimulants et éveillants:
- excitation avec hyper-réactivité chez la souris et le rat,
- augmentation de l'activité motrice spontanée chez la souris avec reprise de l'activité motrice chez la souris habituée à son enceinte, et amélioration de la récupération motrice chez la souris placée en hypoxie aigue,
- augmentation du nombre de passages punis au test des 4 plaques chez la souris,
- diminution de la durée du sommeil barbiturique,
- présence de mouvements stéréotypés chez la souris et le rat, et, potentialisation des stéréotypies induites par l'apomorphine et l'amphétamine chez le rat; et
- une stimulation α- adrénergique périphérique:
- pilo-érection chez la souris et le rat,
- mydriase chez le rat,
- diminution du ptôsis réserpinique chez la souris.

Par ailleurs le CRL 41 245 montre

- un antagonisme total, à forte dose, des effets convulsivants de l'électrochoc,
- une diminution, à forte dose, de l'intensité des tremblements dûs à l'oxotrémorine, et
- une diminution de l'agressivité intergroupes.

En conclusion le CRL 41 245 se présente comme un agent antidépresseur avec une forte composante stimulante.

## I. ESSAIS RELATIFS AU CRL 41 254 (PRODUIT DE L'EXEMPLE 15)

L'étude neuropsychopharmacologique du CRL 41 254 a été effectuée selon les modalités opératoires décrites ci-dessus pour le CRL 41 224.

## I. TOXICITE

Par voie intrapéritonéale chez la souris mâle, le CRL 41 254 présente une DL-0 supérieure à 256 mg/kg et une DL-100 inférieure ou égale à 512 mg/kg.

## II. ACTION SUR LE SNC

Aux fortes doses le CRL 41 254 présente des effets antidépresseurs modestes (antagonisme des hypothermies induites par l'apomorphine et l'oxotrémorine - mais pas d'antagonisme de l'hypothermie réserpinique -), et, des effets stimulants faibles (diminution de la durée du sommeil barbiturique, d'une part, et reprise modérée de l'activité motrice chez la souris habituée à son enceinte, d'autre part).

### III. PROPRIETES IMMUNOLOGIQUES

A la dose de 1 mg/kg, le CRL 41 254 présente une activité immunostimulante statistiquement significative selon le test des cellules formant des plages de lyse après immunisation par les globules rouges de mouton (Test "PFCIgM") décrit par A.J. CUNNINGHAM et al., Immunology 14, pages 599-601 (1968) précité; et aux doses de 10 à 100 mg/kg per os, il présente une activité immunostimulante statistiquement significative selon la mesure de l'intensité de l'hypersensibilité retardée aux globules rouges de mouton, décrite par T.E. MILLER et al., Journal of the National Cancer Institute, 51 (N°5), pages 1669-1676 (1973) précité.

## J. ESSAIS RELATIFS AU CRL 41 262 (PRODUIT DE L'EXEMPLE 17)

Le CRL 41 262 présente des effets antidépresseurs et stimulants du SNC. Il s'est avéré particulièrement intéressant en tant qu'agent immuno-modulateur en raison de son activité immuno-stimulante aux doses de 0,1 mg/kg, 10 mg/kg et 100 mg/kg selon le test PFCIgM précité, et aux doses de 10 et 100 mg/kg per os selon la mesure précitée de l'intensité de l'hypersensibilité retardée aux globules rouges de mouton.

## K. ESSAIS RELATIFS AU CRL 41 247 (PRODUIT DE L'EXEMPLE 14)

L'étude neuropsychopharmacologique du CRL 41 247 a été effectuée selon les modalités décrites ci-dessus pour le CRL 41 224, le CRL 41 247 à tester étant ensolution dans de l'eau distillée à pH 4,5 - 5.5.

### I. TOXICITE

Par voie intrapéritoneale chez la souris mâle, le CRL 41 247 présente une DL-0 supérieure à 512 mg/kg et une DL-60 de l'ordre de 1000 mg/kg environ.

### II. ACTION SUR LE SNC

En bref le CRL 41 247 présente uniquement aux fortes doses, des effets antidépresseurs discrets objectivés par un antagonisme très modéré des hypothermies induites par l'apomorphine et l'oxotrémorine (mais pas d'antagonisme vis-à-vis de l'hypothermie réserpinique), et par une diminution de la durée d'immobilité de la souris placée en immersion forcée; et des effets stimulants objectivés par une augmentation de l'activité motrice chez la souris après habituation à son enceinte et après hypoxie aigue.

On observe par ailleurs que le CRL 41 247 montre, aux fortes doses, une hypothermie (de -3,3°C, 30 minutes après administration intrapéritonéale de 256 mg/kg de CRL 41 247), une diminution des effets convulsivants de l'électrochoc, et, une potentialisation des stéréotypies amphétaminiques.

### III. PROPRIETES IMMUNOLOGIQUES

On observe que le CRL 41 247 stimule l'activité cellulaire selon la technique de T.E. MILLER et al. précitée.

## L. ESSAIS RELATIFS AUX PRODUITS DES EXEMPLES 1-5

Administrés par voie intrapéritonéale les CRL 41 152 (Exemple 1) CRL 41 177 (Exemple 2), CRL 41 192 (Exemple 3), CRL 41 212 (Exemple 4) et CRL 41 217 (Exemple 5) selon l'invention, présentent tous

(i) des effets stimulants qui se manifestent notamment par

- une excitation avec hyperréactivité chez la souris,
- une augmentation de la motilité spontanée chez la souris,
- une reprise de l'activité motrice chez la souris habituée à son enceinte,
- une amélioration de la récupération motrice chez la souris après hypoxie aigue,
- des mouvements stéréotypés chez le rat,
- un antagonisme du sommeil barbiturique, et
- une potentialisation des stéréotypies induites par l'apomorphine ou l'amphétamine;

(ii) des effets antidépresseurs qui se manifestent notamment par:
- un antagonisme des hypothermies induites par l'apomorphine, l'oxotrémorine ou la réserpine, et
- une diminution de l'immobilité dite de "désespoir";

(iii) des effets pouvant refléter une stimulation adrénergique périphérique (notamment une mydriase, un antagonisme du ptôsis réserpinique et un antagonisme des tremblements provoqués par l'oxotrémorine) alors que d'autres signes de stimulation $\alpha$-adrénergique, tels que la salivation, l'exophtalmie, sont absents.

Les CRL 41 152, CRL 41 177, CRL 41 192, CRL 41 212 et CRL 41 217 se différencient tous des substances amphétaminiques par le fait que les mouvements stéréotypés qu'ils induisent chez le rat ne sont pas supprimés par l'injection préalable d'alpha-méthyltyrosine (qui est une substance de référence bloquant la synthèse des catécholamines, circonstance qui empêche l'apparition des stéréotypies amphétaminiques).

Par ailleurs on note que les CRL 41 152, CRL 41 177, CRL 41 212 et CRL 41 217, à la différence du CRL 41 192 et des substances amphétaminiques, n'augmentent pas la toxicité des souris groupées par rapport à celle des souris isolées.

Enfin les CRL 41 152, CRL 41 192 et CRL 41 212 ont des effets anticonvulsivants vis-à-vis des convulsions provoquées notamment par l'électrochoc, alors que les CRL 41 177 et CRL 41 217 potentialisent les effets convulsivants de l'électrochoc.

En outre lorsque chez la souris mâle le CRL 41 177 est administré par voie gastrique (en solution dans de l'eau distillée sous un volume de 20 ml/kg) on constate que (i) son effet sur la motilité spontanée, et (ii) son interaction avec la barbital sont plus importants que par administration intrapéritonéale.

En clinique le CRL 41 152 s'est avéré être un excellent médicament vis-à-vis des dépressions chez l'homme sous forme de comprimés ou gélules renfermant chacun 5 mg de principe actif, à raison de 2 prises par jour; et le CRL 41 177 s'est également avéré être un excellent médicament antidépresseur sous forme de comprimés ou gélules renfermant chacun 2 mg de principe actif, à raison de 2 à 3 comprimés ou gélules par jour.

**Revendications**

1. Nouveau dérivé de 1-(acétylaminophényl)-2-amino-propanone, caractérisé en ce qu'il est choisi parmi l'ensemble constitué par

a) les composés de formule

$Ar-CO-CH(CH_3)-NR_1R_2$ (I)

dans laquelle,

- $R_1$ représente un groupe alkyle en $C_1$-$C_4$, ou un groupe cycloalkyle en $C_3$-$C_6$;
- $R_2$ représente l'atome d'hydrogène ou un groupe alkyle en $C_1$-$C_4$;
- $R_1$ et $R_2$ considérés ensemble peuvent former avec l'atome d'azote auquel ils sont liés un groupe N-hétérocyclique de 5 à 7 sommets susceptible (i) de comporter un second hétéroatome notamment choisi parmi N, O et S, et (ii) d'être substitué, ledit groupe hétérocyclique $NR_1R_2$ étant notamment choisi parmi l'ensemble constitué par les groupes pyrrolidino, morpholino, thiomorpholino, pipéridino, hexaméthylèneimino, pipérazino, 4-méthyl-pipérazino, 4-($\beta$ -hydroxyéthyl)- pipérazino, 4-phénylpipérazino, 4-(p-chlorophényl)-pipérazino;
- Ar représente un groupe acétylaminophényle de formule

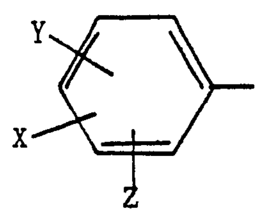

où X est $CH_3CONH$, et, Y et Z qui peuvent être identiques ou différents, représentent chacun un atome d'hydrogène ou d'halogène; et,

b) leurs sels d'addition.

2. Dérivé selon la revendication 1, caractérisé en ce que Ar représente un groupe 4-acétylaminophényle,4-acétylamino-3-chlorophényle, 4-acétylamino-3,5-dichlorophényle, 3-acétylaminophényle ou 3-acétylamino-4-chlorophényle.

3. 1-(4-Acétylamino-3,5-dichlorophényl)-2-isopropylamino-propanone et ses sels d'addition,

4. 1-(4-Acétylaminophényl)-2-éthylamino-propanone et ses sels d'addition.

5. 1-(4-Acétylaminophényl)-2-pipéridino-propanone et ses sels d'addition.

6. 1-(4-Acétylamino-3-chlorophényl)-2-pyrrolidino-propanone et ses sels d'addition.

7. 1-(3-Acétylaminophényl)-2-pipéridino-propanone et ses sels d'addition.

8. 1-(4-Acétylaminophényl)-2-isopropylamino-propanone et ses sels d'addition.

9. 1-(4-Acétylaminophényl)-2-tertiobutylamino-propanone et ses sels d'addition.

10. Composition thérapeutique caractérisée en ce qu'elle renferme en association avec un excipient physiologiquement acceptable, au moins un dérivé de 1-(acétylaminophényl)-2-amino-propanone selon la revendication 1 ou l'un de ses sels d'addition non toxiques.

11. Procédé de préparation d'un dérivé de 1-(acétylaminophényl)-2-amino-propanone selon la revendication 1, caractérisé en ce que l'on fait réagir un dérivé halogéné de formule

$ArCOCH(CH_3)Hal$ (III)

où Ar représente un groupe acétylaminophényle

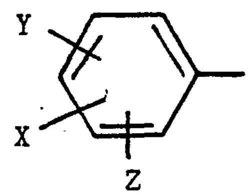

(où X est $CH_3CONH$, Y est H, F, Cl ou Br, Z est H, F, Cl ou Br et Hal représente Cl ou Br) avec une amine de formule

$HNR_1R_2$ (IV)

(où $R_1$ et $R_2$ sont définis comme ci-dessus), à une température comprise entre 15°C et la température de reflux du milieu réactionnel, pendant au moins 0,5 h, à raison de plus d'une mole d'amine IV pour une mole de derivé halogéné III.

12. Procédé de préparation d'un dérivé de 1-(acétylaminophényl)-2-amino-propanone selon la revendication 1, caractérisé en ce que l'on soumet un dérivé de 1-(aminophényl)-2-amino-propanone de formule

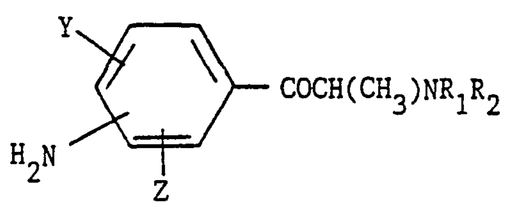

(V)

(où Y, Z, $R_1$ et $R_2$ sont définis comme indiqué ci-dessus) à une réaction d'acétylation au moyen d'un agent d'acétylation en excès par rapport aux conditions stoechiométriques choisi parmi les halogénures d'acétyle et l'anhydride de l'acide acétique, pendant au moins 2 h à la température de reflux du milieu réactionnel.

**Revendications**

1. Procédé de préparation d'un dérivé de 1-(acétylaminophényl)-2-amino-propanone de formule

$Ar\text{-}CO\text{-}CH(CH_3)\text{-}NR_1R_2$ (I)

dans laquelle,

- $R_1$ représente un groupe alkyle en $C_1$-$C_4$, ou un groupe cycloalkyle en $C_3$-$C_6$;
- $R_2$ représente l'atome d'hydrogène ou un groupe alkyle en $C_1$-$C_4$;
- $R_1$ et $R_2$ considérés ensemble peuvent former avec l'atome d'azote auquel ils sont liés un groupe N-

hétérocyclique de 5 à 7 sommets susceptible (i) de comporter un second hétéroatome notamment choisi parmi N, O et S, et (ii) d'être substitué, ledit groupe hétérocyclique $NR_1R_2$ étant notamment choisi parmi l'ensemble constitué par les groupes pyrrolidino, morpholino, thiomorpholino, pipéridino, hexaméthylèneimino, pipérazino, 4-méthyl-pipérazino, 4-(β-hydroxyéthyl)-pipérazino, 4-phénylpipérazino, 4-(p-chlorophényl)-pipérazino;

- Ar représente un groupe acétylaminophényle de formule

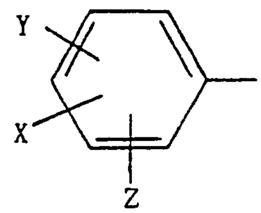

où X est $CH_3CONH$ et, Y et Z qui peuvent être identiques ou différents, représentent chacun un atome d'hydrogène ou d'halogène; et, de ses sels d'addition; ledit procédé étant caractérisé en ce que l'on fait réagir un dérivé halogéné de formule

$ArCOCH(CH_3)Hal$ (III)

où Ar représente un groupe acétylaminophényle

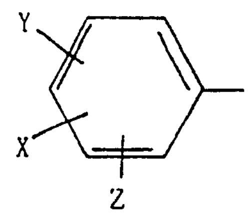

(où X est $CH_3CONH$, Y est H, F, Cl ou Br, Z est H, F,Cl ou Br) et Hal représente Cl ou Br, avec une amine de formule

$HNR_1R_2$ (IV)

(où $R_1$ et $R_2$ sont définis comme ci-dessus), à une température comprise entre 15°C et la température de reflux du milieu réactionnel, pendant au moins 0,5 h, à raison de plus d'une mole d'amine IV pour une mole de dérivé halogéné III.

2. Procédé selon la revendication 1, caractérisé en ce que Ar représente un groupe 4-acétylaminophényle, 4-acétylamino-3-chlorophényle, 4-acétylamino-3,5-dichlorophényle, 3-acétylaminophényle ou 3-acétylamino-4-chlorophényle.

3. Procédé selon la revendication 1, caractérisé en ce que $NR_1R_2$ est un groupe N-hétérocyclique choisi parmi l'ensemble constitué par les groupes pyrrolidino, morpholino, thiomorpholino, pipéridino, 4-méthylpipérazino.

4. Procédé selon la revendication 1, caractérisé en ce que $R_1$ est $CH_3$, $CH_2CH_3$, $CH_2CH_2CH_3$, $CH(CH_3)_2$, $C(CH_3)_3$, ou cyclopropyle et $R_2$ est H ou $CH_3$.

5. Procédé de préparation d'un dérivé de 1-(acétylaminophényl)-2-amino-propanone de formule I selon la revendication 1, caractérisé en ce que l'on soumet un dérivé de 1-(aminophényl)-2-amino-propanone de formule

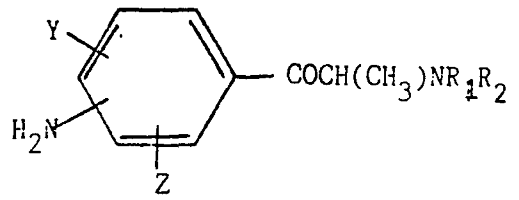

(V)

(où Y, Z, $R_1$ et $R_2$ sont définis comme indique ci-dessus) à une réaction d'acétylation au moyen d'un agent d'acétylation en excès par rapport aux conditions stoechiométriques choisi parmi les halogénures d'acétyle et l'anhydride de l'acide acétique, pendant au moins 2 h à la température de reflux du milieu réactionnel.

6. Procédé selon la revendication 5, caractérisé en ce que Y = Z = Cl.

**Patentansprüche**

1. Neue 1-(Acetylaminophenyl)-2- aminopropanon- Derivate, dadurch gekennzeichnet,
daß sie ausgewählt sind aus
a) den Verbindungen der Formel
Ar-CO-CH($CH_3$)-$NR_1R_2$ (I)
in welcher bedeuten:
$R_1$ eine $C_1$-$C_4$-Alkylgruppe oder eine $C_3$-$C_6$-Cycloal-kylgruppe und $R_2$ H oder eine $C_1$-$C_4$-Alkylgruppe sowie
$R_1$ und $R_2$ zusammen mit dem Stickstoffatom, an welches sie gebunden sind, eine N-heterocyclische Gruppe mit 5 bis 7 Ringatomen bilden können, wobei diese (i) ein zweites Heteroatom ausgewählt aus N, O oder S aufweisen oder (ii) substituiert sein kann, die vorgenannte heterocyclische Gruppe $NR_1R_2$ insbesondere ausgewählt ist aus den Pyrrolidin-, Morpholin-, Thiomorpholin-, Piperidin-, Hexamethylenimino-, Piperazin-, 4-Methylpiperazin-, 4-(β-Hydroxyethyl)-piperazin-, 4-Phenylpiperazin-, 4-(p-Chlorphenyl)-piperazin-Gruppen, Ar einer Acetylaminophenylgruppe der Formel

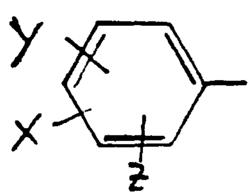

entspricht, in der X $CH_3CONH$ darstellt sowie Y und Z übereinstimmend oder verschieden H oder ein Halogenatom sind, und
b) deren Additionssalzen.

2. Derivat nach Anspruch 1, dadurch gekennzeichnet,
daß Ar eine 4-Acetylaminophenyl-, 4-Acetylamino-3-chlorpenyl-, 4-Acetylamino-3,5-dichlorphenyl-, 3-Acetylaminophenyl- oder 3-Acetylamino-4-chlorphenyl-Gruppe ist.

3. 1-(4-Acetylamino-3,5-dichlorphenyl)-2-isopropylamino-propanon und dessen Additionssalze.

4. 1-(4-Acetylaminophenyl)-2-ethylamino-propanon und dessen Additionssalze.

5. 1-(4-Acetylaminophenyl)-2-piperidino-propanon und dessen Additionssalze.

6. 1-(4-Acetylamino-3-chlorphenyl)-2-pyrrolidino-propanon und dessen Additionssalze.

7. 1-(3-Acetylaminophenyl)-2-piperidino-propanon und dessen Additionssalze.

8. 1-(4-Acetylaminophenyl)-2-isopropylamino-propanon und dessen Additionssalze.

9. 1-(4-Acetylaminophenyl)-2-tert.-butylamino-propanon und dessen Additionssalze.

10. Therapeutische Zusammensetzung, dadurch gekennzeichnet,

daß sie zusammen mit einer physiologisch annehmbaren Grundmasse wenigstens ein Derivat des 1-(Acetylamino-phenyl)-2-aminopropanons gemäß Anspruch 1 oder eines seiner nicht toxischen Additionssalze enthält.

11. Verfahren zur Herstellung von 1-(Acetylaminophenyl)-2-aminopropanon-Derivaten nach Anspruch 1, dadurch gekennzeichnet,
daß man ein Halogenderivat der Formel
$ArCOCH(CH_3)Hal$ (III)
in welcher Ar eine Acetylaminophenylgruppe entsprechend

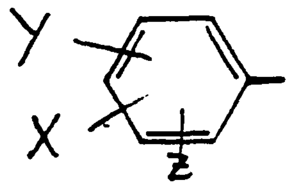

(mit X = $CH_3CONH$, Y = H, F, Cl oder Br und Z = H, F, Cl oder Br) darstellt und Hal, Cl oder Br bedeutet, mit einem Amin der Formel
$HNR_1R_2$ (IV)
(worin $R_1$ und $R_2$ der obigen Bedeutung entsprechen) bei einer Temperatur zwischen 15°C und der Rückflußtemperatur des Reaktionsmediums während mindesten 0,5 Stunden unter Einsatz von mehr als 1 Mol des Amins (IV) je Mol des Halogenderivats (III) miteinander umsetzt.

12. Verfahren zur Herstellung von 1-(Acetylaminophenyl)-2-aminopropanon-Derivaten gemäß Anspruch 1, dadurch gekennzeichnet,
daß man ein Derivat des 1-(Aminophenyl)-2-amino-propanons der Formel

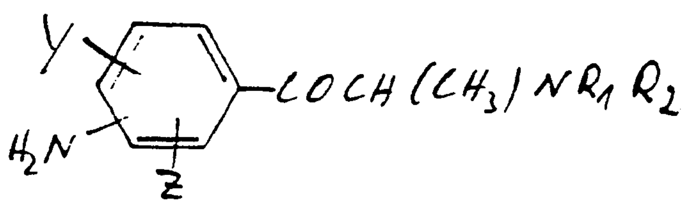

(worin Y, Z, $R_1$ und $R_2$ der obigen Bedeutung entsprechen) einer Acetylierungsreaktion mittels eines im Überschuß angewendeten Acetylierungsmittels, ausgewählt aus Acetylhalogeniden und Essigsäureanhydrid, während mindestens 2 Stunden bei Rückflußtemperatur des Reaktionsmediums unterwirft.

**Patentansprüche**

1. Verfahren zyr Herstellung von 1-(Acetylaminophenyl)-2-amino-propanon-Derivaten der Formel
Ar-CO-CH(CH3)-$NR_1R_2$ (I)
in welcher $R_1$ eine $C_1$-$C_4$-Alkylgruppe oder eine $C_3$-$C_6$-Cycloalkylgruppe bedeutet und $R_2$ H oder eine $C_1$-$C_4$-Alkylgruppe ist sowie $R_1$ und $R_2$ zusammen mit dem Stickstoffatom, an welches sie gebunden sind, eine N-heterocyclische Gruppe mit 5 bis 7 Ringatomen bilden können, wobei diese (i) ein zweites Heteroatom ausgewählt aus N, O oder S aufweisen und (ii) substituiert sein kann, die vorgenannte heterocyclische Gruppe $NR_1R_2$ insbesondere ausgewählt ist aus den Pyrrolidin-Morpholin-. Thiomorpholin-, Piperidin-, Hexamethylenimino-, Piperazin-, 4-Methylpiperazin-, 4-(β-Hydroxyethyl)-piperazin-, 4-Phenylpiperazin-, 4-(p-Chlorphenyl)-piperazin-Gruppen, sowie Ar eine Acetylaminophenylgruppe der Formel

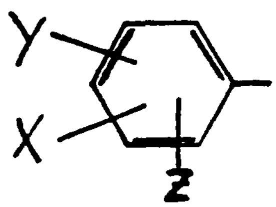

darstellt, in welcher X $CH_3CONH$ entspricht sowie Y und Z übereinstimmend oder verschieden H oder ein Halogenatom sind, sowie deren Additionssalzen, dadurch gekennzeichnet, daß man ein Halogenderivat der Formel

$ArCOCH(CH_3)Hal$ (III)

in welcher Ar eine Acetylaminophenylgruppe der Formel

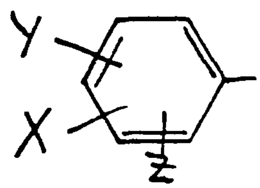

(mit X=$CH_3CONH$, Y=H, F, Cl oder Br und Z = H, F, Cl oder Br) darstellt und Hal Cl oder Br bedeutet, mit einem Amin der Formel

$HNR_1R_2$ (IV)

wobei $R_1$ und $R_2$ der obigen Bedeutung entsprechen, bei einer Temperatur zwischen 15°C und der Rückflußtemperatur des Reaktionsmediums während mindestens 0,5 Stunden unter Einsatz von mehr als 1 Mol des Amins (IV) je Mol des Halogenderivats (III) umsetzt.

2. Verfahren nach Anspruch 1, dadurch gekennzeichnet, daß Ar eine 4-Acetylaminophenyl-, 4-Acetylamino-3-chlorphenyl-, 4-Acetylamino-3,5-dichlorphenyl-, 3-Acetyl-Aminophenyl- oder 3-Acetylamino-4-chlorphenyl-Gruppe darstellt.

3. Verfahren nach Anspruch 1, dadurch gekennzeichnet, daß $NR_1R_2$ eine heterocyclische Gruppe ist, die ausgewählt ist aus den Pyrrolidin-, Morpholin-, Thiomorpholin-, Piperidin- und 4-Methylpiperazin-Gruppen.

4. Verfahren nach Anspruch 1, dadurch gekennzeichnet, daß $R_1$ $CH_3$, $CH_2CH_3$, $CH_2CH_2CH_3$, $CH(CH_3)_2$, $C(CH_3)_3$ oder Cyclopropyl entspricht und $R_2$ H oder $CH_3$ ist.

5. Verfahren zur Herstellung von 1-(Acetylaminophenyl)-2-amino-Propanon-Derivaten der Formel (I) gemäß Anspruch 1, dadurch gekennzeichnet, daß man ein Derivat des 1-(Aminophenyl)-2-aminopropanons der Formel

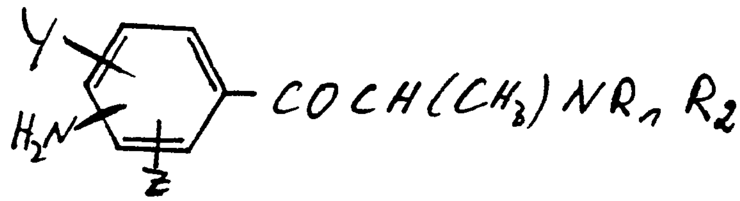

(worin Y, Z, $R_1$ und $R_2$ der obigen Bedeutung entsprechen) einer Acetylierungsreaktion mittels eines im Überschuß angewendeten Acetylierungsmittel, ausgewählt aus Acetylhalogeniden oder Essigsäureanhydrid, während mindestens 2 Stunden bei Rückflußtemperatur des Reaktionsmediums unterwirft.

6. Verfahren nach Anspruch 5, dadurch gekennzeichnet, daß Y = Z = Cl ist.

**Claims**

1. A 1-(acetylaminophenyl)-2-aminopropanone derivative which is selected from the group consisting of

a) the compounds of the formula

$Ar-CO-CH(CH_3)-NR_1R_2$ (I)

in which

- $R_1$ represents a $C_1$-$C_4$ alkyl group or a $C_3$-$C_6$ cycloalkyl group:
- $R_2$ represents the hydrogen atom or a $C_1$-$C_4$ alkyl group;
- $R_1$ and $R_2$, taken together, can form, with the nitrogen atom to which they are bonded, an N-heterocyclic group with 5 to 7 ring members, capable (i) of including a second heteroatom selected especially from N, O and S, and (ii) of being substituted, the said heterocyclic group $NR_1R_2$ being selected especially from the group consisting of pyrrolidino, morpholino, thiomorpholino, piperidino, hexamethyleneimino, piperazino, 4-methylpiperazino, 4-(β-hydroxyethyl)piperazino, 4-phenylpiperazino and 4-(p-chlorophenyl)piperazino groups; and
- Ar represents an acetylaminophenyl group of the formula

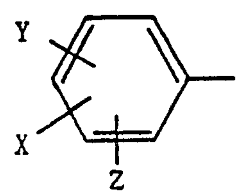

in which X is $CH_3CONH$ and Y and Z, which can be identical or different, each represent a hydrogen or halogen atom; and

b) their addition salts.

2. A derivate as claimed in claim 1, wherein Ar represents a 4-acetylaminophenyl, 4-acetylamino-3-chlorophenyl, 4-acetylamino-3,5-dichlorophenyl, 3-acetylaminophenyl or 3-acetylamino-4-chlorophenyl group.

3. 1-(4-Acetylamino-3,5-dichlorophenyl)-2-isopropylamimo-propanone and its addition salts.

4. 1-(4-Acetylaminophenyl)-2-ethylaminopropanone and its addition salts.

5. 1-(4-Acetylaminophenyl)-2-piperidinopropanone and its addition salts.

6. 1-(4-Acetylamino-3-chlorophenyl)-2-pyrrolidinopropanone and its addition salts.

7. 1-(3-Acetylaminophenyl)-2-piperidinopropanone and its addition salts.

8. 1-(4-Acetylaminophenyl)-2-isopropylaminopropanone and its addition salts.

9. 1-(4-acetylaminophenyl)-2-tert.-butylaminopropanone and its addition salts.

10. A therapeutic composition which contains, in association with a physiologically acceptable excipient, at

least one 1-(acetylaminophenyl)-2-aminopropanone derivative as claimed in claim 1, or one of its non-toxic addition salts.

11. A method for the preparation of a 1-(acetylamino-phenyl)-2-aminopropanone derivative as claimed in claim 1, which comprises reacting a halogen derivative of the formula

$ArCOCH(CH_3)Hal$ (III)

in which Ar represents an acetylaminophenyl group

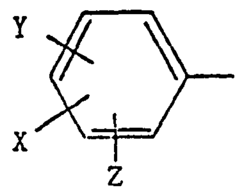

(in which X is $CH_3CONH$, Y is H, F, Cl or Br, Z is H, F, Cl or Br, and Hal represents Cl or Br) with an amine of the formula

$HNR_1R_2$ (IV)

(in which $R_1$ and $R_2$ are defined as above), at a temperature of between 15°C and the reflux temperature of the reaction medium, for at least 0.5 hour, at a rate of more than one mol of amine IV per mol of halogen derivative III.

12. A method for the preparation of a 1-(acetylaminophenyl)-2-aminopropanone derivative as claimed in claim 1, which comprises subjecting a 1-(aminophenyl)-2-aminopropanone derivative of the formula

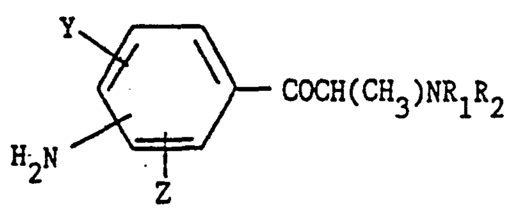

(V)

(in which Y, Z, $R_1$ and $R_2$ are defined as indicated above) to an acetylation reaction by means of an acetylating agent which is in excess relative to the stoichiometric conditions and which is selected from the group consisting of acetylhalides and acetic anhydride, for at least 2 hours, at the reflux temperature of the reaction medium.

**Claims**

1. A method for the preparation of a 1-(acetyl-aminophenyl)-2-aminopropanone derivative of the formula

$Ar-CO-CH(CH_3)-NR_1R_2$ (I)

in which

- $R_1$ represents a $C_1$-$C_4$ alkyl group or a $C_3$-$C_6$ cycloalkyl group;
- $R_2$ represents the hydrogen atom or a $C_1$-$C_4$ alkyl group;
- $R_1$ and $R_2$, taken together, can form, with the nitrogen atom to which they are bonded, an N-heterocyclic group with 5 to 7 ring members, capable (i) of including a second heteroatom selected especially from N, O and S, and (ii) of being substituted, the said heterocyclic group $NR_1R_2$ being selected especially from the group consisting of pyrrolidino, morpholino, thiomorpholino, piperidino, hexamethyleneimino, piperazino, 4-methylpiperazino, 4-(β-hydroxyethyl)piperazino, 4-phenylpiperazino and 4-(p-chlorphenyl)piperazino groups; and
- Ar represents an acetylaminophenyl group of the formula

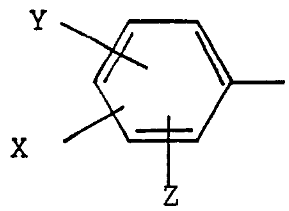

in which X is $CH_3CONH$ and Y and Z, which can be identical or different, each represents a hydrogen or halogen atom; and of its addition salts, said method comprising reacting a halogen derivative of the formula

$ArCOCH(CH_3)Hal$ (III)

in which Ar represents an acetylaminophenyl group

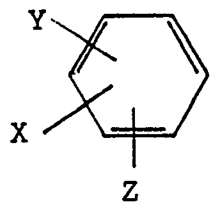

(in which X is $CH_3CONH$, Y is H, F, Cl or Br, Z is H, F, Cl or Br and Hal represents Cl or Br) with an amine of the formula

$HNR_1R_2$ (IV)

(in which $R_1$ and $R_2$ are defined as above), at a temperature of between 15°C and the reflux temperature of the reaction medium, for at least 0.5 hour, at a rate of more than one mol of amine IV per mol of halogen derivative III.

2. A method according to claim 1, wherein Ar represents a 4-acetylaminophenyl, 4-acetylamino-3-chlorophenyl, 4-acetylamino-3,5-dichlorophenyl, 3-acetylaminophenyl or 3-acetylamino-4-chlorophenyl group.

3. A method according to claim 1, wherein $NR_1R_2$ is a heterocyclic group selected from the group consisting of pyrrolidino, morpholino, thiomorpholino, piperidino and 4-methylpiperazino.

4. A method according to claim 1, wherein $R_1$ is $CH_3$, $CH_2CH_3$, $CH_2CH_2CH_3$, $CH(CH_3)_3$ or cyclopropyl and $R_2$ is H or $CH_3$.

5. A method for the preparation of a 1-(acetylamino-phenyl)-2-aminopropanone derivative according to claim 1, which comprises subjecting a 1-(aminophenyl)-2-aminopropanone derivative of the formula

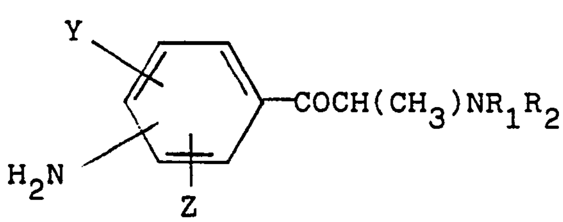

(V)

(in which Y, Z, $R_1$ and $R_2$ are defined as indicated above) to an acetylation reaction by means of an acetylating agent which is in excess relative to the stoichiometric conditions and which is selected from the group consisting of acetyl halides and acetic anhydride, for at least 2 hours, at the reflux temperature of the

reaction medium.

6. A method according to claim 5, wherein Y = Z = Cl.